# EUROPÄISCHE PATENTANMELDUNG

(11) **EP 1 437 144 A1**
(43) Veröffentlichungstag der Anmeldung: **14.07.2004**
(21) Anmeldenummer: 03090007.0
(22) Anmeldetag: 09.01.2003
(51) Int. Cl.: A61K 49/00

(54) **In vivo Transmissible Spongiformen Enzephalopathie Schnelltest im Auge**

(71) Anmelder: IVPT Gmbh, D-16321 Bernau (DE); Lippmann, Corinna, D-14197 Berlin (DE); Lindschau, Carsten, D-14197 Berlin (DE)
(72) Erfinder: Lippmann, Corinna, Dr., 14197 Berlin (DE); Lindschau Carsten, 14197 Berlin (DE)

(57) **Zusammenfassung**

Die Erfindung betrifft den direkten visuellen Nachweis von TSE Erregern im Auge von lebenden Organismen durch Detektion mittels geeigneter Nachweismethoden.

Antikörper/Antikörperfragmente/Aptamere/Farbstoffe werden Fluoreszenz-gekoppelt (z.B. "Alexa 568^{TM}"; "Molecular Probes"). Nach der Aufreinigung werden sie in physiologischer Kochsalzlösung oder anderen adäquaten Formulierungen in das Auge des Tieres (Mensch) appliziert. Bei kranken Tieren kann nach einigen Minuten unter lateraler Illumination, z.B- mit Hilfe eines Laserpointers, eine rötliche Anfärbung des Linsenbereichs wahrgenommen werden, der bei gesunden Tieren nicht vorhanden ist. Bei Verdachtsfällen wird die Diagnose *post mortem* durch weitere Tests validiert.

## Beschreibung

Zu der sehr seltenen Creutzfeldt-Jakob-Krankheit (CJK, CJD), eine irreversible Erkrankung des zentralen Nervensystems beim Menschen, ist 1996 eine in England erstmals beschriebene neue Variante (vCJK, vCJD) hinzugekommen, die auf die Übertragung eines infektiösen Agens durch Nahrungsmittel, die aus an BSE-(Bovine Spongiforme Enzephalopathie) erkrankten Rindern hergestellt wurden, zurückgeführt wird. In mehreren Experimenten konnte gezeigt werden, dass eine Spezies-übergreifende Übertragung möglich ist. Der vCJK sowie weiteren Krankheiten des zentralen Nervensystems [spezifische Creutzfeldt-Jakob-Krankheit (CJK), Gerstmann-Sträussler-Scheinker-Syndrom (GSS), letale familiäre Insomnie (FFI) - Kuru] ist gemeinsam, dass sie pathologische Proteinablagerungen von sogenannten Prion-Proteinen in bestimmten Arealen des Gehirns aufweisen und immer tödlich enden. Zudem gibt es bei verschiedenen Tieren ähnliche Krankheitsbilder [u. a. Chronic Wasting Disease (CWD) der Wapitis, Weißwedel-, Schwarzwedel- und Großohrhirsche (Maultierhirsch) in Nordamerika, Scrapie beim Schaf, die Transmissible Enzephalopathie der Nerze sowie BSE beim Rind (DeArmond SJ, Bouzamondo E. Fundamentals of prion biology and diseases. Toxicology 2002; 181-182:9-16)]. Allgemein werden die hier beschriebenen Erkrankungen unter dem Oberbegriff "TSE" (Transmissible Spongioforme Enzephalopathie) zusammengefasst.

Das übertragbare Agens besteht nach der Prion-Hypothese [Prusiner SB. Novel proteinaceous infectious particles cause scrapie. Science 1982; 216(4542):136-44] aus einer infektiösen, fehlgefalteten Form eines endogenen Proteins (PrP^{C}), dem Prionprotein (PrP^{Sc}), das primär auf neuronalen und lymphatischen Zellen exprimiert wird. Die Vermehrung und Infektiosität des Erregers erfolgt durch Umwandlung der normalen Struktur des Prionproteins in die fehlgefaltete Form, so sind z. B. Mausstämme, denen das Prionprotein-Gen fehlt, experimentell nicht infizierbar. Da diese Krankheitsform zuerst bei Schafen erkannt wurde, wird der TSE-Erreger auch häufig als Scrapie-Isoform des Prionproteins bezeichnet. TSE-Erreger weisen ungewöhnliche Eigenschaften auf. Die Hitzeresistenz übertrifft die von bakteriellen Sporen deutlich, weshalb der Erreger bei den Temperaturen, die für die Zubereitung von Speisen verwendet werden, nicht vollständig inaktiviert wird. Die meisten Desinfektionsmittel, UV-Strahlung, Ionisierende Strahlung, inaktivieren den TSE-Erreger ebenfalls nicht oder nicht ausreichend. Des weiteren konnte bisher keine messbare Immunantwort nachgewiesen werden.

Die Durchführung von BSE-Schnelltests bei allen geschlachteten Rindern, die über 30 Monate alt sind, ist seit 6. Dezember 2000 in Deutschland und seit 1. Januar 2001 EU-weit vorgeschrieben. Seit 31. Januar 2001 müssen in Deutschland auch alle Rinder, die bei der Schlachtung älter als 24 Monate sind, getestet werden, wobei "BSE-getestet" nicht gleichbedeutend mit "BSE-frei" ist. Die derzeit verfügbaren Schnelltestverfahren haben noch deutliche Schwachstellen: Aufgrund ihrer Spezifität sind alle bisher angewandten und evaluierten BSE-Tests erst *post mortem* in einem fortgeschrittenen Krankheitsstadium möglich, d. h. in der Regel bei älteren Tieren. Dazu ist ein Gewebsaufschluss notwendig, wodurch zahlreiche Fehlerquellen entstehen (falsche Lagerung, mehrfaches Auftauen und Einfrieren, zu gründliche oder unzureichende Homogenisation etc.) und der entsprechend zeitaufwendig ist (<6 Stunden nach Schlachtung). Dringend erforderlich ist daher ein zuverlässiger schneller Test an lebenden Tieren, um zu vermeiden, dass unnötig viele nicht infizierte Rinder aus Herden mit einem BSE-Fall getötet werden müssen.

Schon recht früh gab es Hinweise auf eine Beteiligung des Auges an Prionenerkrankungen (Fraser H. Neuronal spread of scrapie agent and targeting of lesions within the retino-tectal pathway. Nature 1982; 295:149-50; Hogan RN, Bowman KA, Baringer JR, Prusiner SB. Replication of scrapie prions in hamster eyes precedes retinal degeneration. Ophthalmic Res 1986; 18:230-235), sowohl als Infektionsquelle (Metha JS, Franks WA. The sclera, the prion, and the ophtalmologist. Br J Ophtalmol 2002; 86:587-592) als auch als Target nach experimentellen Infektion in anderen Organen (Buyukmihci N, Goehring-Harmon F, Marsh RF. Retinal degeneration in experimental scrapie after intraperitoneal or subcutaneous inoculation of hamsters. Exp Neurol 1985; 88: 461-466; Forster J, Farquhar C, Fraser J, Somerville R. Immunolocalization of prion protein in scrapie affected rodent retinas. Neuroscience letters 1999; 260:1-4).

Kürzlich gab es mehrere Publikationen, die die Lokalisation von PrP^{Sc} im Auge belegen (Frederikse PH, Ziegler JS Jr, Farnsworth PN, Carper DA. Prion protein expression in mammalian lenses. Current Eye Res 2000; 20:137-143; Wadsworth JDF, Joiner S, Hill AF, Campbell TA, Desbruslais M, Luthert PJ, Collinge J. Tissue distribution of protease resistant prion protein variant Creutzfedt-Jakob disease using a highly sensitive immunoblotting assay. Lancet 2001; 358: 171-180; Head MW, Northcott V, Rennison K, Ritchie D, McCardle L, Bunn TJ, McLennan NF, Ironside JW, Tullo AB, Bonshek RE. Prion protein accumulation in eyes of patients with sporadic and variant Creutzfeldt-Jakob disease. Invest Ophthalmol Vis Sci 2003; 44: 342-346), wobei in allen Studien mit immunohistologischen Methoden kein zelluläres PrP^{c} nachgewiesen wurde. Die Problematik besteht darin, dass die Erkennung von denaturiertem Protein wie im Western Blot nicht identisch ist zum nativen Protein *in situ.* Die meisten beschriebenen Antikörper sind zur Identifizierung von sowohl PrP^{c} als auch PrP^{Sc} unter denaturierenden Bedingungen geeignet, nicht jedoch für das native, membranverankerte PrP. Der Antikörper 6H4 (Hersteller "Prionics"; nur *"human"* und *"hamster")* und 12F10 (Hersteller "Cayman Chemical"; *"human"* und *"bovine")* sowie der von uns entwickelte GPR10-Antikörper (Abbildung 1; Dot Blot) sind auch zur Erkennung von nativem PrP^{Sc} geignet (Kovacs GG, Head MW, Hegyi I, Bunn TJ, Flicker H, Hainfeller JA, McCardle L, Laszlo L, Jarius C, Ironside JW, Path FRC, Budka H. Immunohistochemistry for the prion protein; Comparison of different monoclonal antibodies in human prion disease subtypes. Brain Pathol 2002; 12: 1-11). 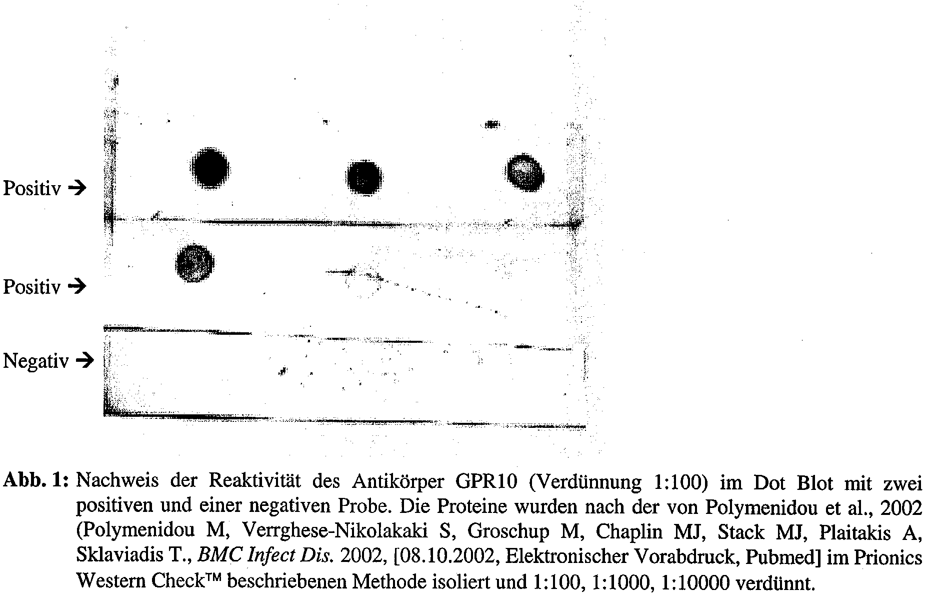 Bisher gibt es keine routinemäßig einsetzbaren Testverfahren, mit denen frühzeitig eine gesicherte Diagnose am lebenden Tier oder beim Menschen gestellt werden kann. Das hier angemeldet Patent ermöglicht einen auf Antikörpern/Antikörperfragmenten/Aptameren/Farbstoffen basierenden hochspezifischen Schnelltest zur Detektion von TSE-Erkrankungen am lebenden Tier und Mensch. Das an einen (Fluoreszenz-)Farbstoff gekoppelte diagnostische Agens wird dazu direkt auf/in das Auge appliziert. Nach hochspezifischer Bindung an die pathologische Form des Prion-Proteins ist die Visualisierung durch adäquate Beleuchtungsquellen und Filtergläser oder andere optische Messsysteme direkt und ohne weitere invasive Eingriffe *in situ* möglich. Das Verfahren ermöglicht eine regelmässige Routinetestung direkt am Standort im Stall durch den Veterinärmediziner und nicht erst im Schlachthof, wodurch eine frühzeitige Erkennung und Separation befallener Tiere möglich ist. Mit dieser Methode kann gegebenenfalls auch beim Menschen TSE diagnostiziert werden, bevor massive neurologische Symptome auftreten, weshalb eine in der Zukunft mögliche Therapie rechtzeitig begonnen werden kann.

### Durchführung:

Die Antikörper/Antikörperfragmente/Aptamere/Farbstoffe werden Fluoreszenz-gekoppelt (z.B. "Alexa 568™"; "Molecular Probes"). Nach der Aufreinigung werden sie in physiologischer Kochsalzlösung oder anderen adäquaten Formulierungen in das Auge des Tieres (Mensch) appliziert. Bei kranken Tieren kann nach einigen Minuten unter lateraler Illumination, z.B- mit Hilfe eines Laserpointers, eine rötliche Anfärbung des Linsenbereichs wahrgenommen werden, der bei gesunden Tieren nicht vorhanden ist. Bei Verdachtsfällen wird die Diagnose *post mortem* durch weitere Tests validiert.

## Patentansprüche

1. Verfahren zur Sichtbarmachung von spezifischen, mit TSE-Erkrankungen [Transmissible Spongioformen Enzephalopathie; z. B. beim Tier: Scrapie, BSE, TSE d. Nerze, CWD d. Hirsche; z. B. beim Menschen: Kuru, CJK, vCJK, GSS, FFI] assoziierten Proteinen im Auge von lebenden Tieren und Menschen durch hochspezifische Farbstoffgekoppelte, und/oder Fluoreszenzfarbstoff-gekoppelte, und/oder enzymgekoppelte Agenzien wie Antikörper, Antikörper-Fab-Fragmente, Aptamere und/oder niedermolekulare Substanzen, gegebenenfalls unter Einbeziehung eines zweiten, fluoreszenz-, und/oder enzym-markierten Antikörpers, und/oder sekundären Antikörpers zur Signalverstärkung, bestehend aus folgenden Schritten in der notwendigen Kombination:
a) Einträufeln, Auftragung mittels Tupfer, invasives Einbringen oder andere Formen der Applikation des diagnostischen Agens in das Auge des Organismus in physiologischer Kochsalzlösung oder anderen adäquaten Formulierungen;
b) gegebenenfalls Applikation [wie unter (a) beschrieben] eines sekundären Antikörpers zur Signalverstärkung;
c) Sichtbarmachung der am und im Auge gebundenen, markierten Agenzien unter Zuhilfenahme von für die verwendeten (Fluoreszenz-) und/oder Farbstoffe, und/oder Enzyme geeigneten Einrichtungen [z. B. Beleuchtungseinrichtungen (Laser o.ä.) und Betrachtung durch Filterbrillen/Augenspiegel mit Filtern oder anderen adäquat ausgestatteten optischen Messsystemen (Kamera, Spektralphotound/oder -fluorimeter o.ä.)].
